(19) Europäisches Patentamt
European Patent Office
Office européen des brevets

(11) EP 2 933 276 B1

(12) EUROPEAN PATENT SPECIFICATION

(45) Date of publication and mention of the grant of the patent:
**09.12.2020 Bulletin 2020/50**

(51) Int Cl.:
***C08F 210/16** (2006.01)* ***C08F 4/659** (2006.01)*

(21) Application number: **14165142.2**

(22) Date of filing: **17.04.2014**

# (54) Improved catalyst system for producing polyethylene copolymers in a high temperature solution polymerization process

Verbessertes Katalysatorsystem zur Herstellung von Polyethylen-Copolymeren in einem Hochtemperatur-Lösungspolymerisationsverfahren

Système de catalyseur amélioré de production de copolymères de polyéthylène dans un processus de polymérisation en solution à température élevée

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**21.10.2015 Bulletin 2015/43**

(73) Proprietor: **Borealis AG**
**1220 Vienna (AT)**

(72) Inventors:
- **Ajellal, Noureddine**
  **00970 Helsinki (FI)**
- **Pellecchia, Roberta**
  **4040 Linz (AT)**
- **Resconi, Luigi**
  **44100 Ferrara (IT)**
- **Izmer, Vyatcheslav V**
  **117186 Moscow (RU)**
- **Kononovich, Dmitry S.**
  **119234 Moscow (RU)**
- **Voskoboynikov, Alexander Z.**
  **129515 Moscow (RU)**

(74) Representative: **Dehns**
**St. Bride's House**
**10 Salisbury Square**
**London EC4Y 8JD (GB)**

(56) References cited:
**WO-A1-00/09515** **WO-A1-97/14727**
**WO-A2-03/051934** **WO-A2-2011/135005**
**WO-A2-2012/001052**

- **DATABASE CA [Online] CHEMICAL ABSTRACTS SERVICE, COLUMBUS, OHIO, US; CHUKANOVA, O. M. ET AL: "Polymerization of propylene using isospecific rac-Me2Si(2-Me,4-PhInd)2ZrCl2 catalyst immobilized on polyethylene with grafted poly(acrylic acid)", XP002729674, retrieved from STN Database accession no. 2001:779046**
- **WANG W-J ET AL: "LONG CHAIN BRANCHING IN ETHYLENE POLYMERIZATION USING CONSTRAINED GEOMETRY METALLOCENE CATALYST", MACROMOLECULAR CHEMISTRY AND PHYSICS, WILEY-VCH VERLAG, WEINHEIM, DE, vol. 199, no. 11, 1 November 1998 (1998-11-01), pages 2409-2416, XP000802214, ISSN: 1022-1352**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 933 276 B1

Printed by Jouve, 75001 PARIS (FR)

## Description

The present invention is related to improved catalyst systems, which are able to produce polyethylene copolymers in a high temperature solution polymerization process. The catalyst systems comprise a combination of selected bisindenyl metallocene complexes, substituted at least in position 2 and 4 of both indenyls along with cocatalyst mixture comprising an aluminoxane cocatalyst and a boron based cocatalyst. These combinations remarkably give rise to catalyst systems with excellent activity, productivity and stability and allow production of polyethylene copolymers with increased comonomer incorporation.

Metallocene catalysts have been used to manufacture polyolefins for many years. Countless academic and patent publications describe the use of these catalysts in olefin polymerization. Metallocenes are now used industrially and polyethylenes and in particular polypropylenes are often produced using cyclopentadienyl based catalyst systems with different substitution patterns.

Several of these metallocene catalysts have been described for the use in solution polymerization in particular for producing polypropylene.

For example WO 2007/116034 describes i.a. a catalyst system comprising racemic dimethylsilylbis(2-methyl-4-phenyl-5-methoxy-6-tert-butylinden-1-yl)dichlorozirconium and methylalumoxane cocatalyst for producing polypropylene in a solution polymerization process at temperatures between 100°C and 120°C.
It is mentioned that the metallocene compounds can also be used for preparing ethylene copolymers, preferably ethylene-butene copolymers, but it is said that such copolymers are obtained by using gas phase processes.
Also WO 2007/122098 describes the use of the complex racemic dimethylsilylbis(2-methyl-4-(4-tert-butylphenyl)-1,5,6,7-tetrahydro-s-indacen-1-yl)dichlorozirconium in combination with an alumoxane cocatalyst for producing ethylene copolymers at 100°C.

EP 2532687 A describes further metallocene complexes, like dimethylsilanediylbis[2-methyl-4-(3,5-di-*tert*-butylphenyl)-7-methoxy-indenyl]zirconiumdichloride, which is first pre-alkylated with an aluminium alkyl compound and then activated with borate cocatalyst. The catalyst system is used for preparing polypropylene at a temperature between 30°C to 70°C.

WO 2011/135004 complexes as described in WO 2007/116034, like racemic dimethylsilylbis(2-methyl-4-phenyl-5-methoxy-6-tert-butylinden-1-yl)dichlorozirconium and prepared according to the emulsion/solidification method as described in WO 2003/051934 are disclosed. These complexes are activated with an alumoxane cocatalyst and used for propylene polymerization.

WO 2012/075560 further describes a multi stage (at least two stage) solution polymerization process for preparing ethylene copolymers, wherein a phosphinimine catalyst is used with a cocatalyst comprising an alkylaluminoxane and an ionic activator, like a boron compound.

WO2011/135005 discloses catalyst systems containing metallocene catalysts comprising bis indenyl $\pi$-ligands and a co-catalyst for the polymerisation of polypropylene.

Although a lot of work has been done in the field of metallocene catalysts, there still remain some problems, which relate especially to the productivity or activity of the catalyst systems when used in a high temperature solution polymerization process. The productivity or activity has been found to be relatively low.

There remains a need therefore to find new catalyst systems for ethylene copolymerization in a high temperature solution polymerization process, which are able to produce the ethylene copolymers with desired properties and which have high activity and/or productivity.

The present inventors have now found improved catalyst systems, which are able to solve the problems disclosed above. In particular, the invention combines the use of special metallocene complexes with a cocatalyst mixture comprising aluminoxane cocatalysts and a boron based cocatalyst in a high temperature solution polymerization process for producing ethylene copolymers.

## Summary of Invention

Thus, viewed from one aspect the invention relates to a catalyst system for producing ethylene copolymers in a high temperature solution process, the catalyst system comprising

(i) a metallocene complex of formula (I)

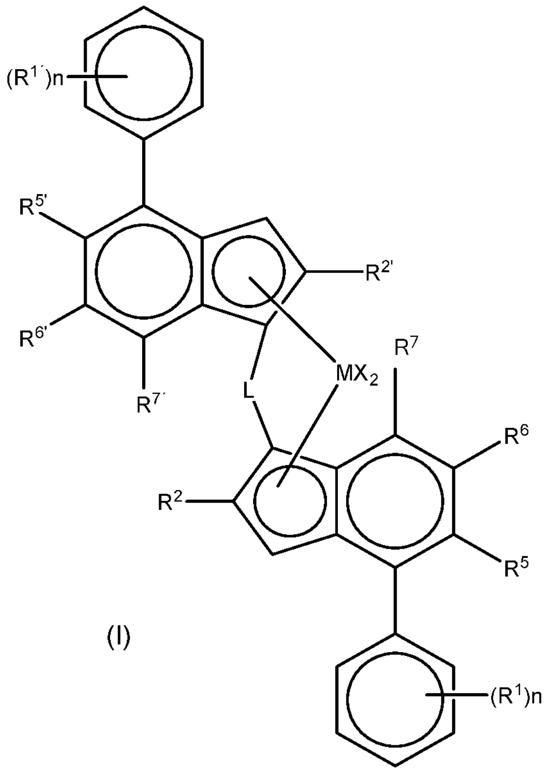

(I)

wherein

M is Hf or Zr,
X is a sigma ligand,
L is a bridge of the formula $-SiR^8_2-$, wherein each $R^8$ is independently a $C_1$-$C_{20}$-hydrocarbyl, tri($C_1$-$C_{20}$-alkyl)silyl, $C_6$-$C_{20}$-aryl, $C_7$-$C_{20}$-arylalkyl or $C_7$-$C_{20}$-alkylaryl,
n is 0, 1 or 2,
$R^1$ and $R^{1'}$ are the same or can be different and can be a linear or branched $C_1$-$C_6$-alkyl group,
$R^2$ and $R^{2'}$ are the same or can be different and are a $CH_2$-$R^9$ group, with $R^9$ being H or linear or branched $C_1$-$C_6$-alkyl group,
$R^5$ and $R^{5'}$ are different and one is H and the other is an -OR group, wherein R is a $C_1$-$C_6$-alkyl group,
$R^6$ and $R^{6'}$ are the same or are different and can be H or a $C(R^{10})_3$ group, with $R^{10}$ being the same or different and $R^{10}$ can be H or a linear or branched $C_1$-$C_6$-alkyl group, and
$R^7$ and $R^{7'}$ can be the same or are different and can be H or a linear or branched $C_1$-$C_6$-alkyl group,

(ii) an aluminoxane cocatalyst and
(iii) a boron containing cocatalyst. Viewed from another aspect the invention provides a process for the preparation of an ethylene copolymer comprising polymerizing ethylene and a $C_{4-10}$ alpha-olefin comonomer in a high temperature solution process at a temperature greater than 100°C in the presence of a catalyst comprising:

(i) a metallocene complex of formula (I) as defined above
(ii) an aluminoxane cocatalyst and
(iii) a boron containing cocatalyst.

## Detailed Description of the Invention

### Metallocene Complex

The single site metallocene complex, especially the complexes defined by the formula (I) specified in the present invention, used for manufacture of the ethylene copolymer are symmetrical or asymmetrical. For asymmetrical complexes that means that the two indenyl ligands forming the metallocene complex are different, that is, each indenyl ligand bears a set of substituents that are either chemically different, or located in different positions with respect to the other indenyl

ligand. More precisely, they are chiral, racemic bridged bisindenyl metallocene complexes.

Whilst the complexes of the invention may be in their syn configuration, ideally they are in their anti configuration. For the purpose of this invention, racemic-anti means that the two indenyl ligands are oriented in opposite directions with respect to the cyclopentadienyl-metal-cyclopentadienyl plane, while racemic-syn means that the two indenyl ligands are oriented in the same direction with respect to the cyclopentadienyl-metal-cyclopentadienyl plane, as shown in the Figure below.

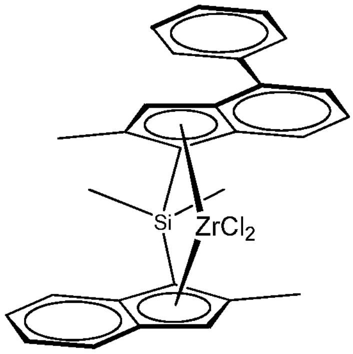

Racemic Anti

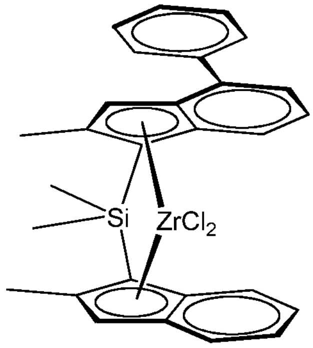

Racemic Syn

Formula (I) is intended to cover both syn and anti configurations.

By nature of their chemistry, both anti and syn enantiomer pairs are formed during the synthesis of the complexes. However, by using the ligands of this invention, separation of the preferred anti isomers from the syn isomers is straightforward.

It is preferred if the metallocene complexes of the invention are employed as the rac anti isomer. Ideally therefore at least 95% mol, such as at least 98% mol, especially at least 99% mol of the metallocene catalyst is in the racemic anti isomeric form.

The invention can be effected with a metallocene complex of formula (I)

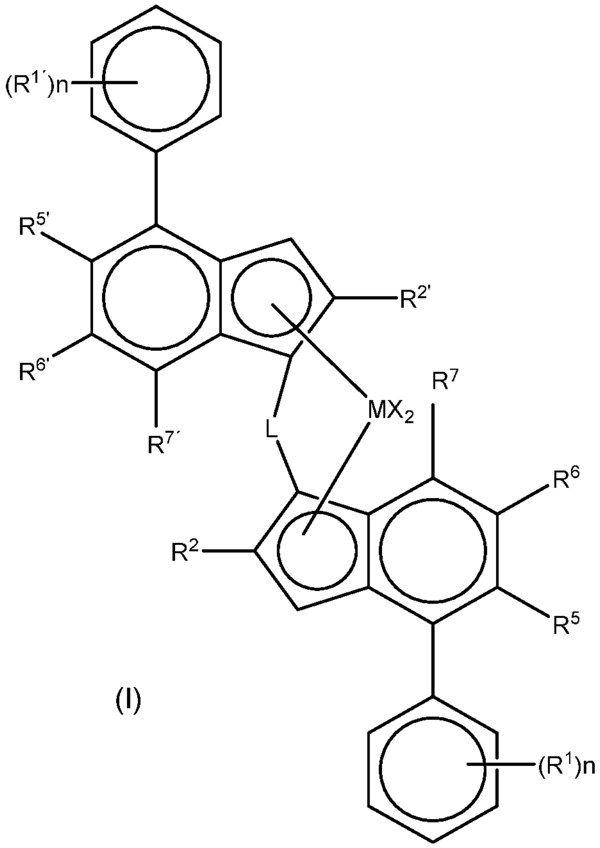

(I)

wherein

M is Hf or Zr,
X is a sigma ligand,
L is a bridge of the formula $-SiR^8_2-$, wherein each $R^8$ is independently a $C_1$-$C_{20}$-hydrocarbyl, tri($C_1$-$C_{20}$-alkyl)silyl, $C_6$-$C_{20}$-aryl, $C_7$-$C_{20}$-arylalkyl or $C_7$-$C_{20}$-alkylaryl
n is 0, 1 or 2,
$R^1$ and $R^{1'}$ are the same or can be different and can be a linear or branched $C_1$-$C_6$-alkyl group,
$R^2$ and $R^{2'}$ are the same or can be different and are a $CH_2$-$R^9$ group, with $R^9$ being H or linear or branched $C_1$-$C_6$-alkyl group,
$R^5$ and $R^{5'}$ are different and one is H and the other is an -OR group, wherein R is a $C_1$-$C_6$-alkyl group,
$R^6$ and $R^{6'}$ are the same or are different and can be H or a $C(R^{10})_3$ group, with $R^{10}$ being the same or different and $R^{10}$ can be H or a linear or branched $C_1$-$C_6$-alkyl group, and
$R^7$ and $R^{7'}$ can be the same or are different and can be H or a linear or branched $C_1$-$C_6$-alkyl group.

In the formula (I) each X, which may be the same or different, is a sigma ligand, preferably a hydrogen atom, a halogen atom, a $R^{11}$, $OR^{11}$, $OSO_2CF_3$, $OCOR^{11}$, $SR^{11}$, $NR^{11}_2$ or $PR^{11}_2$ group wherein $R^{11}$ is a linear or branched, cyclic or acyclic, $C_1$-$C_{20}$-alkyl, $C_2$-$C_{20}$-alkenyl, $C_2$-$C_{20}$-alkynyl, $C_6$-$C_{20}$-aryl, $C_7$-$C_{20}$-alkylaryl or $C_7$-$C_{20}$-arylalkyl radical; optionally containing heteroatoms belonging to groups 14-16 or is $SiR^{11}_3$, $SiHR^{11}_2$ or $SiH_2R^{11}$. $R^{11}$ is preferably a $C_{1-6}$-alkyl, phenyl or benzyl group, whereby the term halogen includes fluoro, chloro, bromo and iodo groups, preferably chloro groups.
More preferably each X is independently a hydrogen atom, a halogen atom, $C_{1-6}$-alkoxy group or an $R^{11}$ group, e.g. preferably a $C_{1-6}$-alkyl, phenyl or benzyl group.
Most preferably X is chlorine or a methyl radical. Preferably both X groups are the same.
n is 0, 1 or 2
$R^1$ and $R^{1'}$ can be a linear or branched $C_1$-$C_6$-alkyl group, like methyl, ethyl, n-propyl, i-propyl, n-butyl or tert.-butyl. Preferably $R^1$ and $R^{1'}$ are the same and are a linear or branched $C_1$-$C_6$-alkyl group, more preferably a linear or branched $C_2$-$C_6$-alkyl group, more preferably a linear or branched butyl-group and most preferably $R^1$ and $R^{1'}$ are tert.-butyl.
In a preferred embodiment at least one of the phenyl groups is substituted with at least one of $R^1$ or $R^{1'}$, thus n can be

0 only for one of the ligands.

If n is 1, then $R^1$ and $R^{1'}$ are preferably on position 4 (para) of the phenyl ring and if n is 2 then $R^1$ and $R^{1'}$ are preferably on positions 3 and 5 of the phenyl ring.

Different combinations for $R^1$ and $R^{1'}$ are possible:

Both phenyl rings are substituted by $R^1$ and $R^{1'}$, whereby n can be the same or can be different for the two phenyl rings and is 1 or 2.

Only one phenyl ring is substituted, whereby n is 1 or 2, preferably 1.

$R^2$ and $R^{2'}$ are the same or can be different and are a $CH_2$-$R^9$ group, with $R^9$ being H or linear or branched $C_1$-$C_6$-alkyl group, like methyl, ethyl, n-propyl, i-propyl, n-butyl, i-butyl, sec.-butyl and tert.-butyl. Preferably $R^2$ and $R^{2'}$ are the same and are a $CH_2$-$R^8$ group, with $R^9$ being H or linear or branched $C_1$-$C_4$-alkyl group, more preferably $R^2$ and $R^{2'}$ are the same and are a $CH_2$-$R^9$ group, with $R^9$ being H or linear or branched $C_1$-$C_3$-alkyl group and most preferably $R^2$ and $R^{2'}$ are either both methyl or both i-butyl.

$R^5$ and $R^{5'}$ are different and one is H and the other is an -OR group, wherein R is a linear or branched $C_1$-$C_6$-alkyl group, and $R^6$ and $R^{6'}$ are the same or are different and can be a H or a $C(R^{10})_3$ group, with $R^{10}$ being the same or different and $R^{10}$ can be H or a linear or branched $C_1$-$C_6$-alkyl group.

It is also possible that at both ligands $R^5$ and $R^6$ as well as $R^5$ and $R^{6'}$ are hydrogen. A further possibility is that only one of the ligands is unsubstituted in position 5 and 6.

$R^7$ and $R^{7'}$ can be the same or are different and can be H or a linear or branched $C_1$-$C_6$-alkyl group, preferably $R^7$ and $R^{7'}$ are the same or are different and can be H or a linear or branched $C_1$-$C_4$-alkyl group and more preferably $R^7$ and $R^{7'}$ are the same or are different and can be H or a $C_1$-$C_2$-alkyl group.

For preferred complexes $R^7$ and $R^{7'}$ are the same and are both H,

or for a further class of preferred complexes one of $R^7$ or $R^{7'}$ is a linear or branched $C_1$-$C_6$-alkyl group, preferably a linear or branched $C_1$-$C_4$-alkyl group and more preferably a $C_1$-$C_2$-alkyl group and the other is H.

L is a bridge of the formula $-SiR^8_2-$, wherein each $R^8$ is independently a $C_1$-$C_{20}$-hydrocarbyl, tri($C_1$-$C_{20}$-alkyl)silyl, $C_6$-$C_{20}$-aryl, $C_7$-$C_{20}$-arylalkyl or $C_7$-$C_{20}$-alkylaryl.

The term $C_{1-20}$ hydrocarbyl group therefore includes $C_{1-20}$ alkyl, $C_{2-20}$ alkenyl, $C_{2-20}$ alkynyl, $C_{3-20}$ cycloalkyl, $C_{3-20}$ cycloalkenyl, $C_{6-20}$ aryl groups, $C_{7-20}$ alkylaryl groups or $C_{7-20}$ arylalkyl groups or of course mixtures of these groups such as cycloalkyl substituted by alkyl.

Unless otherwise stated, preferred $C_{1-20}$ hydrocarbyl groups are $C_{1-20}$ alkyl, $C_{4-20}$ cycloalkyl, $C_{5-20}$ cycloalkyl-alkyl groups, $C_{7-20}$ alkylaryl groups, $C_{7-20}$ arylalkyl groups or $C_{6-20}$ aryl groups. Preferably $R^8$ are the same and are a $C_1$-$C_{10}$-hydrocarbyl or $C_6$-$C_{10}$-aryl group, like methyl, ethyl, propyl, isopropyl, tertbutyl, isobutyl, $C_{5-6}$-cycloalkyl, cyclohexylmethyl, phenyl or benzyl, more preferably both $R^8$ are a $C_1$-$C_4$-hydrocarbyl or $C_6$-aryl group and most preferably both $R^8$ are a $C_1$-alkyl group.

Especially preferred complexes of formula (I) are

*racemic* dimethylsilyl[(2-methyl-4-phenyl-5-methoxy-6-tert-butylinden-1-yl)-(2-methyl-4-phenyl-inden-1-yl)] zirconium dichloride or dimethyl,

either in their syn or anti configuration.

For the purpose of this invention, the terms dimethylsilyl, dimethylsilanediyl and dimethylsilylene are equivalent.

## Synthesis

The ligands required to form the catalysts of the invention can be synthesised by any process and the skilled organic chemist would be able to devise various synthetic protocols for the manufacture of the necessary ligand materials. WO2007/116034 discloses the necessary chemistry. Synthetic protocols can also generally be found in WO2002/02576, WO2011/135004, WO2012/084961, WO2012/001052 and WO2011/076780.

## Cocatalyst

To form an active catalytic species it is normally necessary to employ a cocatalyst as is well known in the art. The present invention requires the use of an aluminoxane cocatalyst and an additional boron containing cocatalyst.

The aluminoxane cocatalyst can be one of formula (II):

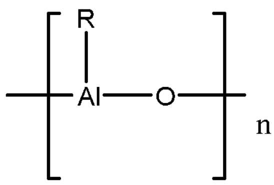

(II)

where n is usually from 6 to 20 and R has the meaning below.

Aluminoxanes are formed on partial hydrolysis of organoaluminum compounds, for example those of the formula $AlR_3$, $AlR_2Y$ and $Al_2R_3Y_3$ where R can be, for example, C1-C10 alkyl, preferably C1-C5 alkyl, or C3-10-cycloalkyl, C7-C12 -arylalkyl or alkylaryl and/or phenyl or naphthyl, and where Y can be hydrogen, halogen, preferably chlorine or bromine, or C1-C10 alkoxy, preferably methoxy or ethoxy. The resulting oxygen-containing aluminoxanes are not in general pure compounds but mixtures of oligomers of the formula (I).

The preferred aluminoxane in the process according to the invention is methylaluminoxane (MAO). Since the aluminoxanes used according to the invention as cocatalysts are not, owing to their mode of preparation, pure compounds, the molarity of aluminoxane solutions hereinafter is based on their aluminium content.

It has been surprisingly found however, that in the context of heterogeneous catalysis, where catalysts are not supported on any external carrier or supported as described above, that higher activities can be achieved if a boron based cocatalyst is also employed as a cocatalyst. It will be appreciated by the skilled man that where boron based cocatalysts are employed, it is normal to preactivate the complex by reaction thereof with an aluminium alkyl compound, such as TIBA. This procedure is well known and any suitable aluminium alkyl, preferably an aluminium alkyl compounds of the formula (VIII) $AlR_3$ with R being a linear or branched $C_2$-$C_8$-alkyl group, can be used.

Preferred aluminium alkyl compounds are triethylaluminium, tri-isobutylaluminium, tri-isohexylaluminium, tri-n-octylaluminium and tri-isooctylaluminium.

The present invention combines the use of boron cocatalysts with aluminoxanes rather than the combination of these simple aluminium alkyls and boron cocatalysts.

Boron based cocatalysts of interest include boron compounds containing a borate $3^+$ ion, i.e. borate compounds. These compounds generally contain an anion of formula:

$(Z)_4B^-$ (III)

where Z is an optionally substituted phenyl derivative, said substituent being a halo-$C_{1-6}$-alkyl or halo group. Preferred options are fluoro or trifluoromethyl. Most preferably, the phenyl group is perfluorinated.

Such ionic cocatalysts preferably contain a non-coordinating anion such as tetrakis(pentafluorophenyl)borate.

Suitable counterions are protonated amine or aniline derivatives or phosphonium ions. These may have the general formula (IV) or (V):

$NQ_4^+$ (IV) or $PQ_4^+$ (V)

where Q is independently H, $C_{1-6}$-alkyl, $C_{3-8}$ cycloakyl, phenyl$C_{1-6}$-alkylene- or optionally substituted Ph. Optional substituents may be C1-6-alkyl, halo or nitro. There may be one or more than one such substituent. Preferred substituted Ph groups include therefore para-substituted phenyl, preferably tolyl or dimethylphenyl.

It is preferred if at least one Q group is H, thus preferred compounds are those of formula:

$NHQ_3^+$ (VI) or $PHQ_3^+$ (VII)

Preferred phenyl-$C_{1-6}$-alkyl- groups include benzyl.

Suitable counterions therefore include: methylammonium, anilinium, dimethylammonium, diethylammonium, N-methylanilinium, diphenylammonium, N,N-dimethylanilinium, trimethylammonium, triethylammonium, tri-n-butylammonium, methyldiphenylammonium, p-bromo-N,N- dimethylanilinium or p-nitro-N,N-dimethylanilinium, especially dimethylammonium or N,N-dimethylanilinium. The use of pyridinium as an ion is a further option.

Phosphonium ions of interest include triphenylphosphonium, triethylphosphonium, diphenylphosphonium, tri(methylphenyl)phosphonium and tri(dimethylphenyl)phosphonium. A more preferred counterion is trityl ($CPh_3^+$) or analogues thereof in which the Ph group is functionalised to carry one or more alkyl groups. Highly preferred borates of use in the invention therefore comprise the tetrakis(pentafluorophenyl)borate ion.

Preferred ionic compounds which can be used according to the present invention include: tributylammoniumtetra(pentafluorophenyl)borate,

tributylammoniumtetra(trifluoromethylphenyl)borate,
tributylammoniumtetra-(4-fluorophenyl)borate,
N,N-dimethylcyclohexylammoniumtetrakis-(pentafluorophenyl)borate,
N,N-dimethylbenzylammoniumtetrakis(pentafluorophenyl)borate,
N,N-dimethylaniliniumtetrakis(pentafluorophenyl)borate,
N,N- di(propyl)ammoniumtetrakis(pentafluorophenyl)borate,
di(cyclohexyl)ammoniumtetrakis(pentafluorophenyl)borate,
triphenylcarbeniumtetrakis(pentafluorophenyl)borate,
or ferroceniumtetrakis(pentafluorophenyl)borate.

Preference is given to triphenylcarbeniumtetrakis(pentafluorophenyl) borate,
N,N- dimethylcyclohexylammoniumtetrakis(pentafluorophenyl)borate,
N,N- dimethylbenzylammoniumtetrakis(pentafluorophenyl)borate or
N,N-dimethylaniliniumtetrakis(pentafluorophenyl)borate.

It has been surprisingly found that certain boron cocatalysts are especially preferred. Preferred borates of use in the invention therefore comprise the trityl ion. Thus the use of N,N-dimethylammonium-tetrakispentafluorophenylborate and $Ph_3CB(PhF_5)_4$ and analogues therefore are especially favoured.

It is also possible to add an aluminium alkyl compounds of the formula (VIII) $AlR_3$ with R being a linear or branched $C_2$-$C_8$-alkyl group as acid scavengers in amounts known to the art skilled person.

Suitable amounts of cocatalyst will be well known to the skilled man.

The molar ratio of boron to the metal ion of the metallocene may be in the range 0.5:1 to 10:1 mol/mol, preferably 1:1 to 10:1, especially 1:1 to 5:1 mol/mol.
The molar ratio of Al in the aluminoxane to the metal ion of the metallocene may be in the range 1:1 to 2000:1 mol/mol, preferably 10:1 to 1000:1, and more preferably 50:1 to 500:1 mol/mol.

**Catalyst Manufacture**

The metallocene complex of the present invention is used in combination with the cocatalysts as a catalyst system for the polymerization of ethylene and $C_{4-10}$ alpha-olefin comonomer in a high temperature solution polymerization process.

The catalyst system of the invention can be used in non-supported form or in solid form. The catalyst system of the invention may be used as a homogeneous catalyst or heterogeneous catalyst.
The catalyst system of the invention in solid form, preferably in solid particulate form is free from an external carrier, however still being in solid form.
By free from an external carrier is meant that the catalyst does not contain an external support, such as an inorganic support, for example, silica or alumina, or an organic polymeric support material.

**a) Non-supported**

Non-supported catalyst systems, suitable for the present invention can be prepared in solution, for example in an aromatic solvent like toluene, by contacting the metallocene (as a solid or as a solution) with the cocatalyst(s), for example methylaluminoxane and/or a borane or a borate salt previously in an aromatic solvent, or can be prepared by sequentially adding the dissolved catalyst components to the polymerization medium.

**b) Solid form**

In an alternative embodiment, in order to provide the catalyst system of the invention in solid form but without using an external carrier, it is preferred if a liquid/liquid emulsion system is used. The process involves forming dispersing catalyst components (i) (the complex) and (ii) + optionally (iii) the cocatalysts in a solvent, and solidifying said dispersed droplets to form solid particles.

In the present case, where aluminoxan as well as boron based cocatalysts are used, it is particularly preferred if the aluminoxane is contacted with the metallocene before the borate is added. Both cocatalyst components and the metallocene are preferably present in one solution.

In particular, the method involves preparing a solution of the catalyst components; dispersing said solution in an solvent to form an emulsion in which said one or more catalyst components are present in the droplets of the dispersed phase; immobilising the catalyst components in the dispersed droplets, in the absence of an external particulate porous support, to form solid particles comprising the said catalyst, and optionally recovering said particles.
This process enables the manufacture of active catalyst particles with improved morphology, e.g. with a predetermined particle size, spherical shape, compact structure, excellent surface properties and without using any added external

porous support material, such as an inorganic oxide, e.g. silica. The catalyst particles can have a smooth surface, they may be compact in nature and catalyst active components can be distributed uniformly thorough the catalyst particles.

Full disclosure of the necessary process steps can be found in WO03/051934.

All or part of the preparation steps can be done in a continuous manner. Reference is made to WO2006/069733 describing principles of such a continuous or semicontinuous preparation methods of the solid catalyst types, prepared via emulsion/solidification method.

The formed catalyst preferably has good stability/kinetics in terms of longevity of reaction, high activity and the catalysts enable low ash contents.

The use of the heterogeneous, non-supported catalysts, (i.e. "self-supported" catalysts) might have, as a drawback, a tendency to dissolve to some extent in the polymerization media, i.e. some active catalyst components might leach out of the catalyst particles during slurry polymerization, whereby the original good morphology of the catalyst might be lost.

These leached catalyst components are very active possibly causing problems during polymerization. Therefore, the amount of leached components should be minimized, i.e. all catalyst components should be kept in heterogeneous form.

Furthermore, the self-supported catalysts generate, due to the high amount of catalytically active species in the catalyst system, high temperatures at the beginning of the polymerization which may cause melting of the product material. Both effects, i.e. the partial dissolving of the catalyst system and the heat generation, might cause fouling, sheeting and deterioration of the polymer material morphology.

In order to minimise the possible problems associated with high activity or leaching, it is preferred to "prepolymerize" the catalyst before using it in polymerization process. It has to be noted that prepolymerization in this regard is part of the catalyst preparation process, being a step carried out after a solid catalyst is formed. This catalyst prepolymerization step is not part of the actual polymerization configuration, which might comprise a conventional process prepolymerization step as well. After the catalyst prepolymerization step, a solid catalyst is obtained and used in polymerization.

Catalyst "prepolymerization" takes place following the solidification step of the liquid-liquid emulsion process hereinbefore described. Prepolymerization may take place by known methods described in the art, such as that described in WO 2010/052263, WO 2010/052260 or WO 2010/052264. Preferable embodiments of this aspect of the invention are described herein.

Use of the catalyst prepolymerization step offers the advantage of minimising leaching of catalyst components and thus local overheating.

**Polymer**

The polymer to be produced using the catalyst system of the invention is copolymer of ethylene and a $C_{4-10}$ alpha-olefin comonomer, like 1-butene, 1-hexene, 4-methyl-1-pentene, and 1-octene. Preferably butene, hexene or octene and more preferably octene is used as comonomer.

The comonomer content in such a polymer may be up to 40 wt%, preferably between 5 to 40 wt%, more preferably 10 to 38 wt% and more preferable 15 to 36 wt%.

The density (measured according to ISO 1183-187) of the polymers is in the range of 0.850 g/cm$^3$ to 0.950 g/cm$^3$, preferably in the range of 0.850 g/cm$^3$ to 0.945 g/cm$^3$ and more preferably in the range of 0.850 g/cm$^3$ to 0.940 g/cm$^3$.

Mw/Mn value of the polymers of the invention is less than 5, e.g. in the range of 2.0 to 4.5.

The melting points (measured with DSC according to ISO 11357-3:1999) of the polymers to be produced are below 130°C, preferably below 120°C, more preferably below 110°C and most preferably below 100°C

**Polymerization**

The catalyst system of the present invention is used to produce the above defined ethylene copolymers in a high temperature solution polymerization process at temperatures higher than 100°C.

In view of this invention such process is essentially based on polymerizing the monomer and a suitable comonomer in a liquid hydrocarbon solvent in which the resulting polymer is soluble. The polymerization is carried out at a temperature above the melting point of the polymer, as a result of which a polymer solution is obtained. This solution is flashed in order to separate the polymer from the unreacted monomer and the solvent. The solvent is then recovered and recycled in the process.

A solution polymerization process is known for its short reactor residence times (compared to Gas-phase or slurry processes) allowing, thus, very fast grade transitions and significant flexibility in producing a wide product range in a short production cycle.

According to the present invention the used solution polymerization process is a high temperature solution polymerization process, using a polymerization temperature of higher than 100°C. Preferably the polymerization temperature is at least 110°, more preferably at least 150°C. The polymerization temperature can be up to 250°C.

The pressure in the used solution polymerization process according to the invention is preferably in a range of 10 to 100 bar [1 to 10 MPa], preferably 15 to 100 bar [1.5 to 10 MPa] and more preferably 20 to 100 bar [2 to 10 MPa]. The liquid hydrocarbon solvent used is preferably a $C_{5-12}$-hydrocarbon, which may be unsubstituted or substituted by $C_{1-4}$ alkyl group, such as pentane, methyl pentane, hexane, heptane, octane, cyclohexane, methylcyclohexane and hydrogenated naphtha. More preferably unsubstituted $C_{6-10}$-hydrocarbon solvents are used.

A known solution technology suitable for the process according to the invention is the COMPACT technology.

**Advantage**

The new catalyst systems, comprising component (i), (ii) and optionally (iii) can be advantageously used for ethylene copolymerization in high temperature solution polymerization process.
The catalyst systems according to the present invention show excellent productivity, excellent comonomer incorporation and thermal stability if used for ethylene copolymerization in high temperature solution polymerization process.

**Applications**

The polymers made by the catalyst system of the invention are useful in all kinds of end articles such as pipes, films (cast, blown films), fibers, moulded articles (e.g. injection moulded, blow moulded, rotomoulded articles), extrusion coatings and so on.

The invention will now be illustrated by reference to the following non-limiting examples

**Examples:**

**Methods**

**Al and Zr determination (ICP-method)**

The elemental analysis of a catalyst was performed by taking a solid sample of mass, m. The catalyst was deactivated by substituting the inert storing conditions with ambient air, first passively through a needle and then actively by applying vacuum three times to the sampling container. Samples were dissolved to a volume V by first cooling on dry ice while adding freshly deionised water (5% of V) and nitric acid ($HNO_3$, 65 %, 5 % of V). The samples were transferred in full to volumetric flasks using deionised water and rinsing the sampling containers. Hydrofluoric acid (HF, 40 %, 3 % of V) was added to the volumetric flasks and volume V obtained by addition of freshly deionised water. The prepared sample solutions were left to stabilise for two hours.

The analysis was run at room temperature using a Thermo Elemental iCAP 6300 Inductively Coupled Plasma - Optical Emission Spectrometer (ICP-OES) which was calibrated using a blank (a solution of 5 % HNO3, 5 % HF in deionised water), and 6 standards of 0.5 ppm, 1 ppm, 10 ppm, 50 ppm, 100 ppm and 300 ppm of Al, with 0.5 ppm, 1 ppm, 5 ppm, 20 ppm, 50 ppm and 100 ppm of B and P in solutions of 5 % HNO3, 3 % HF in deionised water. Immediately before analysis the calibration is 'resloped' using the blank and 100 ppm Al, 50 ppm B, P standard, a quality control sample (20 ppm Al, 5 ppm B, P in a solution of 5 % HNO3, 3 % HF in DI water) is run to confirm the reslope. The QC sample is also run after every 5th sample and at the end of a scheduled analysis set.

The reported values are an average of three successive aliquots taken from the same sample and are related back to the original catalyst by inputting the original mass of sample, m, and the dilution volume, V, into the software.

**DSC analysis**

The melting point ($T_m$) and crystallization temperature ($T_c$) were determined on a DSC200 TA instrument, by placing a 5-7 mg polymer sample, into a closed DSC aluminum pan, heating the sample from -30 °C to 180 °C at 10 °C/min, holding for 5 min at 180 °C, cooling from 180 °C to -30 °C, holding for 5 min at -30 °C, heating from -30 °C to 180 °C at 10 °C/min. The reported $T_m$ is the maximum of the curve from the second heating scan and $T_c$ is the maximum of the curve of the cooling scan.

**Quantification of comonomer content by NMR spectroscopy**

Quantitative nuclear-magnetic resonance (NMR) spectroscopy was used to quantify the comonomer content

of the polymers.

Quantitative $^{13}C\{^1H\}$ NMR spectra recorded in the molten-state using a Bruker Advance III 500 NMR spectrometer operating at 500.13 and 125.76 MHz for $^1H$ and $^{13}C$ respectively. All spectra were recorded using a $^{13}C$ optimised 7 mm magic-angle spinning (MAS) probehead at 150°C using nitrogen gas for all pneumatics. Approximately 200 mg of material was packed into a 7 mm outer diameter zirconia MAS rotor and spun at 4 kHz. This setup was chosen primarily for the high sensitivity needed for rapid identification and accurate quantification.{klimke06, parkinson07, castignolles09, parkinson11} Standard single-pulse excitation was employed utilising the transient NOE at short recycle delays of 3s {pollard04, klimke06} and the RS-HEPT decoupling scheme{fillip05,griffin07}. A total of 1024 (1k) transients were acquired per spectrum. This setup was chosen due its high sensitivity towards low comonomer contents.

Quantitative $^{13}C\{^1H\}$ NMR spectra were processed, integrated and quantitative properties determined using custom spectral analysis automation programs. All chemical shifts are internally referenced to the bulk methylene signal ($\delta$+) at 30.00 ppm {randall89}.

Characteristic signals corresponding to the incorporation of 1-octene were observed (randall89, liu01, qiu07) and all comonomer contents calculated with respect to all other monomers present in the polymer.

Characteristic signals resulting from isolated 1-octene incorporation i.e. EEOEE comonomer sequences, were observed. Isolated 1-octene incorporation was quantified using the integral of the signal at 38.32 ppm. This integral is assigned to the unresolved signals corresponding to both $_*$B6 and $_*\beta$B6B6 sites of isolated (EEOEE) and isolated double non-consecutive (EEOEOEE) 1-octene sequences respectively. To compensate for the influence of the two $_*\beta$B6B6 sites the integral of the $\beta\beta$B6B6 site at 24.7 ppm is used:

$$O = I_{*B6+*\beta B6B6} - 2 * I_{\beta\beta B6B6}$$

Characteristic signals resulting from consecutive 1-octene incorporation, i.e. EEOOEE comonomer sequences, were also observed. Such consecutive 1-octene incorporation was quantified using the integral of the signal at 40.48 ppm assigned to the $\alpha\alpha$B6B6 sites accounting for the number of reporting sites per comonomer:

$$OO = 2 * I_{\alpha\alpha B6B6}$$

Characteristic signals resulting from isolated non-consecutive 1-octene incorporation, i.e. EEOEOEE comonomer sequences, were also observed. Such isolated non-consecutive 1-octene incorporation was quantified using the integral of the signal at 24.7 ppm assigned to the $\beta\beta$B6B6 sites accounting for the number of reporting sites per comonomer:

$$OEO = 2 * I_{\beta\beta B6B6}$$

Characteristic signals resulting from isolated triple-consecutive 1-octene incorporation, i.e. EEOOOEE comonomer sequences, were also observed. Such isolated triple-consecutive 1-octene incorporation was quantified using the integral of the signal at 41.2 ppm assigned to the $\alpha\alpha\gamma$B6B6B6 sites accounting for the number of reporting sites per comonomer:

$$OOO = 3/2 * I_{\alpha\alpha\gamma B6B6B6}$$

With no other signals indicative of other comonomer sequences observed the total 1-octene comonomer content was calculated based solely on the amount of isolated (EEOEE), isolated double-consecutive (EEOOEE), isolated non-consecutive (EEOEOEE) and isolated triple-consecutive (EEOOOEE) 1-octene comonomer sequences:

$$O_{total} = O + OO + OEO + OOO$$

Characteristic signals resulting from saturated end-groups were observed. Such saturated end-groups were quantified using the average integral of the two resolved signals at 22.84 and 32.23 ppm. The 22.84 ppm integral is assigned to the unresolved signals corresponding to both 2B6 and 2S sites of 1-octene and the saturated chain end respectively. The 32.23 ppm integral is assigned to the unresolved signals corresponding to both 3B6 and 3S sites of 1-octene and the saturated chain end respectively. To compensate for the influence of the 2B6 and 3B6 1-octene sites

the total 1-octene content is used:

$$S = (1/2)*( I_{2S+2B6} + I_{3S+3B6} - 2*O_{total})$$

The ethylene comonomer content was quantified using the integral of the bulk methylene (bulk) signals at 30.00 ppm. This integral included the $\gamma$ and 4B6 sites from 1-octene as well as the $\delta^+$ sites. The total ethylene comonomer content was calculated based on the bulk integral and compensating for the observed 1-octene sequences and end-groups:

$$E_{total} = (1/2)*[ I_{bulk} + 2*O + 1*OO + 3*OEO + 0*OOO + 3*S ]$$

It should be noted that compensation of the bulk integral for the presence of isolated triple-incorporation (EEOOOEE) 1-octene sequences is not required as the number of under and over accounted ethylene units is equal.

The total mole fraction of 1-octene in the polymer was then calculated as:

$$fO = ( O_{total} / ( E_{total} + O_{total} )$$

The total comonomer incorporation of 1-octene in weight percent was calculated from the mole fraction in the standard manner:

$$O [wt\%] = 100 * ( fO * 112.21) / ( (fO * 112.21) + ((1-fO) * 28.05) )$$

klimke06
Klimke, K., Parkinson, M., Piel, C., Kaminsky, W., Spiess, H.W., Wilhelm, M., Macromol. Chem. Phys. 2006;207:382.

parkinson07
Parkinson, M., Klimke, K., Spiess, H.W., Wilhelm, M., Macromol. Chem. Phys. 2007;208:2128.

parkinson11
NMR Spectroscopy of Polymers: Innovative Strategies for Complex Macromolecules, Chapter 24, 401 (2011)

pollard04
Pollard, M., Klimke, K., Graf, R., Spiess, H.W., Wilhelm, M., Sperber, O., Piel, C., Kaminsky, W., Macromolecules 2004;37:813.

filip05
Filip, X., Tripon, C., Filip, C., J. Mag. Resn. 2005, 176, 239

griffin07
Griffin, J.M., Tripon, C., Samoson, A., Filip, C., and Brown, S.P., Mag. Res. in Chem. 2007 45, S1, S198

castignolles09
Castignolles, P., Graf, R., Parkinson, M., Wilhelm, M., Gaborieau, M., Polymer 50 (2009) 2373

zhou07
Zhou, Z., Kuemmerle, R., Qiu, X., Redwine, D., Cong, R., Taha, A., Baugh, D. Winniford, B., J. Mag. Reson. 187 (2007) 225

busico07
Busico, V., Carbonniere, P., Cipullo, R., Pellecchia, R., Severn, J., Talarico, G., Macromol. Rapid Commun. 2007, 28, 1128

randall89

J. Randall, Macromol. Sci., Rev. Macromol. Chem. Phys. 1989, C29, 201.

qui07
Qiu, X., Redwine, D., Gobbi, G., Nuamthanom, A., Rinaldi, P., Macromolecules 2007, 40, 6879

liu01
Liu, W., Rinaldi, P., McIntosh, L., Quirk, P., Macromolecules 2001, 34, 4757

GPC: Molecular weight averages, molecular weight distribution, and polydispersity index ($M_n$, $M_w$, $M_w/M_n$) Molecular weight averages (Mw, Mn), Molecular weight distribution (MWD) and its broadness, described by polydispersity index, PDI= Mw/Mn (wherein Mn is the number average molecular weight and Mw is the weight average molecular weight) were determined by Gel Permeation Chromatography (GPC) according to ISO 16014-4:2003 and ASTM D 6474-99. A Waters GPCV2000 instrument, equipped with differential refractive index detector and online viscosimeter was used with 2 x GMHXL-HT and 1x G7000HXL-HT TSK-gel columns from Tosoh Bioscience and 1,2,4-trichlorobenzene (TCB, stabilized with 250 mg/L 2,6-Di tert butyl-4-methyl-phenol) as solvent at 140 °C and at a constant flow rate of 1 mL/min. 209.5 μL of sample solution were injected per analysis. The column set was calibrated using universal calibration (according to ISO 16014-2:2003) with at least 15 narrow MWD polystyrene (PS) standards in the range of 1 kg/mol to 12 000 kg/mol. Mark Houwink constants for PS, PE and PP used are as per ASTM D 6474-99. All samples were prepared by dissolving 0.5 - 4.0 mg of polymer in 4 mL (at 140 °C) of stabilized TCB (same as mobile phase) and keeping for max. 3 hours at max. 160°C with continuous gentle shaking prior sampling into the GPC instrument.

**Chemicals**

MAO was purchased from Chemtura and used as a 30 wt-% solution in toluene.
Triphenylcarbeniumtetrakis(pentafluorophenyl)borate (alternative name trityl tetrakis-(pentafluorophenyl)borate) (CAS 136040-19-2) was purchased from Acros (tritylBF20)
1-octene as co-monomer (99%, Sigma Aldrich) was dried over molecular sieves and degassed with nitrogen before use.
Heptane and decane (99.9 %, Sigma Aldrich) were dried under molecular sieves and degassed with nitrogen before use.

**Examples:**

For the purpose of this invention, the terms dimethylsilyl, dimethylsilanediyl and dimethylsililene are equivalent.

**Complex preparation**

**1. Complex 1-Zr:** anti-dimethylsilylene(2-methyl-4-phenyl-5-methoxy-6-*tert*-butyl-indenyl)(2-methyl-4-(4-*tert*-butyl-phenyl)indenyl)zirconium dichloride (C1-Zr) was prepared as described in the patent application WO2013/007650A1

**b) Catalyst system**

**Comparative Example 1**

Rac Complex 1-Zr was used for preparing Comparative Catalyst system CCS-1
C1-Zr has been prepared following the procedure described in WO 2013/007650 A1 for catalyst E2, by adjusting the metallocene and MAO amounts in order to achieve the Al/Zr ratios indicated in table 1. The complex has been off-line prepolymerized with propylene, following the procedure described in WO 2013/007650 A1 for catalyst E2P.
The Degree of off-line pre-polymerization 3.3 g/g
Al/Zr molar ratio in catalyst 431 mol/mol
Metallocene complex content of off-line prepolymerized catalyst 0.696 wt.%

**Inventive Example 1:**

Rac Complex 1-Zr was used for preparing Inventive Catalyst system ICS-1

Step 1.

Inside the glovebox, 87.90 mg of Rac C1-Zr, prepared as described above was mixed with 4 ml of 30 wt.-% Chemtura MAO in a septum bottle and the solution was stirred for 60 minutes and then 105.2 mg of trityl tetrakis(pentafluorophenyl)borate was added. The mixture was left to react overnight at room temperature inside the glovebox. Then, in another septum bottle, 54 μL of dry and degassed FluorN 474 was mixed with 2 mL of 30 wt.-% Chemtura MAO. The solutions were left under stirring overnight.

The following day, 4 mL of the MAO-metallocene-borate solution and 1 mL of the surfactant-MAO solution were successively added into a 50mL emulsification glass reactor containing 40mL of PFC at -10 °C and equipped with an overhead stirrer (stirring speed = 600 rpm). Total amount of MAO is 5 mL (200 equivalents). A red emulsion formed immediately (measured emulsion stability = 19 seconds) and stirred during 15 minutes at -10 °C / 600rpm.

Then the emulsion was transferred via a 2/4 teflon tube to 100mL of hot PFC at 90 °C and stirred at 600 rpm until the transfer is completed. Then the speed was reduced to 300 rpm. After 15 minutes stirring, the oil bath was removed and the stirrer turned off. The catalyst was left to settle up on top of the PFC and after 35 minutes the solvent was siphoned off. The remaining red catalyst was dried during 2 hours at 50 °C over an argon flow. 0.86 g of a red free flowing powder was obtained (Al-wt% = 31.2 and Zr-wt% = 0.49).

*Off-line prepolymerization procedure*

Off-line pre-polymerization experiment was done in a 125 mL pressure reactor equipped with gas-feeding lines and an overhead stirrer. Dry and degassed perfluoro-1.3-dimethylcyclohexane (15 $cm^3$) and 0.6855 g of the catalyst produced in the step 1, to be pre-polymerized, were loaded into the reactor inside a glove box and the reactor was sealed. The reactor was then taken out from the glove box and placed inside a water cooled bath kept at 25 °C. The overhead stirrer and the feeding lines were connected and stirring speed set to 450 rpm. The experiment was started by opening the propylene feed into the reactor. The total pressure in the reactor was raised to about 5 bar [0.5 MPa] and held constant by propylene feed via mass flow controller until the target degree of polymerization was reached (DP ≈ 4.0). The reaction was stopped by flashing the volatile components. Inside glove box, the reactor was opened and the content poured into a glass vessel. The perfluoro-1.3-dimethylcyclohexane was evaporated until a constant weight was obtained to yield 3.42 g of the pre-polymerized ICS-1 catalyst.

**Inventive Example 2:**

Rac Complex 1-Zr was used for preparing Inventive Catalyst system ICS-2

Inside the glovebox, 88.03 mg of complex 1-Zr was mixed with 5 ml MAO in a septum bottle and the solution was stirred for 60 minutes and then 105.15 mg of tritylBF20 was added. The mixture was left to react overnight at room temperature inside the glovebox.

**Table 1:** Catalyst System Composition

| cat. | Metallocene | DofP[1] | Al/Zr[2] | B/Zr[3] |
|---|---|---|---|---|
| | | [g/g] | [mol/mol] | [mol/mol] |
| CE-1 | C1-Zr | 3.3 | 431 | 0.0 |
| IE-1 | C1-Zr | 4.0 | 215 | 1.0 |
| IE-2 | C1-Zr | n.a | 200 | 1.0 |

[1]Degree of off-line pre-polymerization
[2] Al/Zr molar ratio in catalyst
[3] Al/Zr molar ratio in catalyst

n.a not applicable

**Polymerization**

In order to prove the suitability of the catalyst systems according to the present invention two kind of polymerizations were performed.

In Examples IE-1 and CE-1 the polymerization reaction were carried out in a 480 mL pressure reactor at 110°C.

In Example IE-2 the polymerization reaction was carried out in Parallel Polymerization Reactors (PPR) (provided by Symyx) (10 mL Reactor Volume) at 190°C

**Polymerization procedure IE-1 and CE-1:**

**The catalyst system ICS-1 was used and as Comparative Example the catalyst system CCS-1 was used (both prepared as described above)**

Ethylene/1-octene solution polymerizations was performed according to the following procedure in heptane at 110°C without $H_2$.

First 1-octene was fed into the reactor by means of a Waters HPLC pump in the desired amounts, then 200 mL heptane by means of 10 mL syringe. The stirring speed was set to 150 rpm. 50 μmol of triethylaluminium (TEA) (as a scavenger) as a 0.5 moL/L solution in heptane was fed into the reactor. The reactor temperature was set to 110°C. The reactor was fed with ethylene up to the required pressure (P = 20 bar [2 MPa]) and the desired catalyst amount was injected at the polymerization temperature. The pressure was kept constant, feeding ethylene and after 20min polymerization time the catalyst was killed by air addition and venting the reactors. The samples were stabilized with 2500 ppm Irganox B225 (dissolved in acetone).

**Table 2:** results for ethylene/1-octene solution co-polymerization

| Example | IE-1 | CE-1 |
|---|---|---|
| Used Catalyst System | ICS-1* | CCS-1* |
| Cat amount [mg] | 3.5 | 20.0 |
| Used 1-octene [g] | 19 | 12 |
| Yield [g] | 0.75 | 0.50 |
| Productivity in 20 min [kg/g MC] | 17.0 | 3.6 |
| Ethylene/1-octene in liq. Phase [wt/wt] | 0.44 | 0.76 |
| 1-Octene incorporation [wt% NMR] | 14.4 | n.m. |
| MWD | 3.5 | n.m. |
| *prepolymerized<br>n.m. not measured | | |

**PPR polymerization procedure and characterisations for IE-2**

**Pre-catalyst preparation procedure (ternary system MC/MAO/tritylBF20):**

Inside a glovebox, desired amount of metallocene was mixed with 5 ml MAO solution in a septum bottle and the solution was stirred for 60 minutes and then 105.15 mg of tritylBF20 was added. The mixture was left to react overnight at room temperature inside the glovebox. All catalysts were prepared according to the below recipe (Table 3).

**Table 3:** pre-catalyst preparation of the selected metallocenes.

| Example | IE-2 |
|---|---|
| C1-Zr [mg] | 88.03 |
| MAO [mg] | 1320 |
| TritylBF20 [mg] | 105.15 |
| Al/Zr | 200 |
| B/Zr | 1.0 |

MAO was used as 30% solution in toluene

**Polymerization procedure for PPR:**

The selected catalysts were screened at 190°C, with polymerization solvent decane, at one MAO/Zr ratio (200), one B/Zr ratio (1.0) and 1-octene/ethylene ratios of 1 wt/wt ($C_8/C_2$ = 1.0 wt/wt).

Stock solutions of the metallocenes and co-catalysts (MAO and Borate) were prepared to be used for each set of reactions.

The vessels were loaded inside a glovebox utilizing a 3-axis liquid handling robot. A preweighed glass vial with stirring paddles was sealed and purged with nitrogen. A volume of about 4 mL of corresponding solvent (decane) was filled in each PPR reactor. Then, adequate amount of triethyl aluminium (TEA) as scavenger was added, along with precise volume of octene as co-monomer at room temperature. The ethylene pressure was set to 10 bar [1 MPa] to check any leaks. Then, the temperature and pressure had been increased to the set value (T = 190°C and 24 bar [2.4 MPa]) and once the steady state was reached, the corresponding volume of pre-activated catalyst as a slurry in toluene had been injected in the reactor to start the polymerization under mechanical stirring. The run was quenched with $CO_2$ after the set amount of ethylene uptake had been reached (20 min as a maximum run time).The glass vials had been dried by vacuum centrifuge and weighed.

**Table 4:** PPR experiments conditions for ethylene/1-octene solution co-polymerization

| | ICS-2 |
|---|---|
| Catalyst system [μl] | 51.8 |
| Used 1-octene [g] | 0.45 |
| Decane [g] | 3.14 |
| TEAL Scavenger [μmol] | 15.0 |

**Table 5:** PPR experiments results for ethylene/1-octene solution co-polymerization

| Example | MC | Complex amount [mg] | Quench time [min] | Productivity [gPE/mgCat | Tm [°C] | Tc [°C] | 1-octene incorporation [wt% NMR] |
|---|---|---|---|---|---|---|---|
| IE-2 | Complex 1-Zr | 0.08 | 9.1 | 4.6 | n. m. | n. m. | 24.2 |
| n. m. not measured | | | | | | | |

**Claims**

1. Catalyst system for producing ethylene copolymers in a high temperature solution process, the catalyst system comprising

(i) a metallocene complex of formula (I)

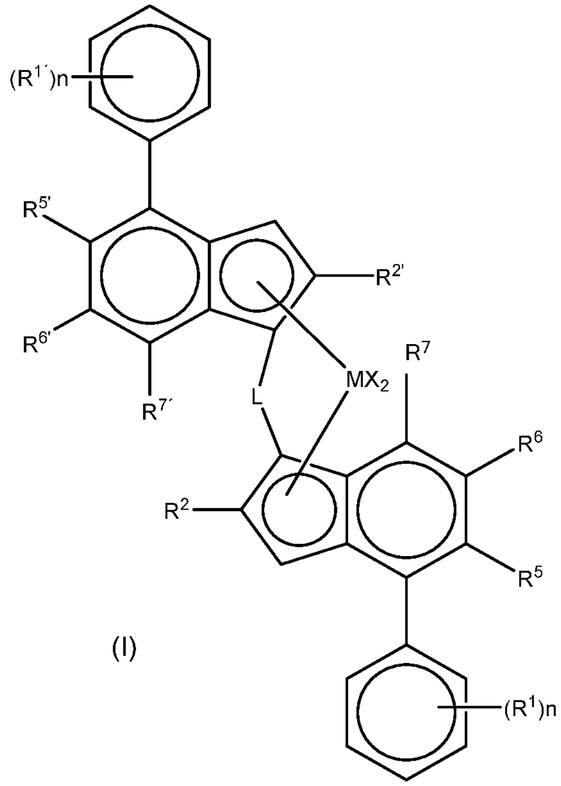

(I)

wherein

M is Hf or Zr,
X is a sigma ligand,
L is a bridge of the formula $-SiR^8_2-$, wherein each $R^8$ is independently a $C_1$-$C_{20}$-hydrocarbyl, tri($C_1$-$C_{20}$-alkyl)silyl, $C_6$-$C_{20}$-aryl, $C_7$-$C_{20}$-arylalkyl or $C_7$-$C_{20}$-alkylaryl,
n is 0, 1 or 2,
$R^1$ and $R^{1'}$ are the same or can be different and can be a linear or branched $C_1$-$C_6$-alkyl group,
$R^2$ and $R^{2'}$ are the same or are different and are a $CH_2$-$R^9$ group, with $R^9$ being H or linear or branched $C_1$-$C_6$-alkyl group,
$R^5$ and $R^{5'}$ are different and one is H and the other is an -OR group, wherein R is a $C_1$-$C_6$-alkyl group;
$R^6$ and $R^{6'}$ are the same or are different and can be H or a $C(R^{10})_3$ group, with $R^{10}$ being the same or different and $R^{10}$ can be H or a linear or branched $C_1$-$C_6$-alkyl group; and
$R^7$ and $R^{7'}$ can be the same or are different and can be H or a linear or branched $C_1$-$C_6$-alkyl group;

(ii) an aluminoxane cocatalyst; and
(iii) a boron containing cocatalyst.

**2.** Catalyst system according to claim 1, wherein in the formula (I)
M is Zr,
X which may be the same or different, and is a hydrogen atom, a halogen atom, a $R^{11}$, $OR^{11}$, $OSO_2CF_3$, $OCOR^{11}$, $SR^{11}$, $NR^{11}_2$ or $PR^{11}_2$ group, wherein $R^{11}$ is a linear or branched, cyclic or acyclic, $C_1$-$C_{20}$-alkyl, $C_2$-$C_{20}$-alkenyl, $C_2$-$C_{20}$-alkynyl, $C_6$-$C_{20}$-aryl, $C_7$-$C_{20}$-alkylaryl or $C_7$-$C_{20}$-arylalkyl radical; optionally containing heteroatoms belonging to groups 14-16 or is $SiR^{11}_3$, $SiHR^{11}_2$ or $SiH_2R^{11}$,L is a bridge of the formula $-SiR^8_2-$, wherein both $R^8$ are the same $C_1$-$C_{10}$-hydrocarbyl or $C_6$-$C_{10}$-aryl group,
$R^1$ and $R^{1'}$ are the same and are a linear or branched $C_1$-$C_6$-alkyl group,
n is 0, 1 or 2, with the proviso that n is not 0 for at least one of the phenyls groups,
$R^2$ and $R^{2'}$ are the same and are a $CH_2$-$R^9$ group, with $R^9$ being H or linear or branched $C_1$-$C_4$-alkyl group,
$R^5$ and $R^{5'}$ are different and one is H and the other is an -OR group, wherein R is a $C_1$-$C_4$-alkyl group,
$R^6$ and $R^{6'}$ are the same or are different and can be H or a $C(R^{10})_3$ group, with $R^{10}$ being the same or different and $R^{10}$ can be a linear or branched $C_1$-$C_4$-alkyl group, and

$R^7$ and $R^{7'}$ can be the same or are different and can be H or a linear or branched $C_1$-$C_4$-alkyl group.

**3.** Catalyst system according to claim 1 wherein in the formula (I)
M is Zr,
X is independently a hydrogen atom, a halogen atom, $C_{1-6}$-alkoxy group or an $R^{11}$ group, wherein $R^{11}$ a $C_{1-6}$-alkyl, phenyl or benzyl group,
L is a bridge of the formula -$SiR^8_2$-, wherein both $R^8$ are the same $C_1$-$C_{10}$-hydrocarbyl or $C_6$-$C_{10}$-aryl group,
$R^1$ and $R^{1'}$ are the same and are a linear or branched $C_1$-$C_6$-alkyl group,
n is 0, 1 or 2, with the proviso that n is 1 for at least one of the phenyls groups,
$R^2$ and $R^{2'}$ are the same and are a $CH_2$-$R^9$ group, with $R^9$ being H or linear or branched $C_1$-$C_4$-alkyl group,
$R^5$ and $R^{5'}$ are different and one is H and the other is an OR group, wherein R is a $C_1$-$C_4$-alkyl group;
$R^6$ and $R^{6'}$ are the same or are different and can be H or a $C(R^{10})_3$ group, with $R^{10}$ being the same or different and $R^{10}$ can be a linear or branched $C_1$-$C_4$-alkyl group, and
$R^7$ and $R^{7'}$ can be the same or are different and can be H or a linear or branched $C_1$-$C_4$-alkyl group

**4.** Catalyst system according to claim 1 wherein the metallocene of formula (I) is selected from
racemic dimethylsilyl[(2-methyl-4-phenyl-5-methoxy-6-tert-butylinden-1-yl)-(2-methyl-4-phenyl-inden-1-yl)] zirconium dichloride or dimethyl,
either in their syn or anti configuration, or
*racemic* anti-dimethylsilyl[2-methyl-4-phenyl-5-methoxy-6-*tert*-butyl-indenyl)(2-methyl-4-(4-*tert*-butyl-phenyl)indenyl)zirconium dichloride.

**5.** Catalyst system according to any of preceding claims, being obtainable by a process in which

(a) a liquid/liquid emulsion system is formed, said liquid/liquid emulsion system comprising a solution of the catalyst components (i) to (iii) dispersed in a solvent so as to form dispersed droplets; and
(b) solid particles are formed by solidifying said dispersed droplets.

**6.** A catalyst system according to claim 5, wherein the solid particles are prepolymerized in a step (c).

**7.** Catalyst system according to any of preceding claims, being a non-supported catalyst systems obtainable by contacting the metallocene of formula (I) as a solid or as a solution with the cocatalyst(s) previously dissolved in an aromatic solvent, or being obtainable by sequentially adding the catalyst components to the polymerization medium.

**8.** A catalyst system as claimed in any preceding claim wherein said aluminoxane cocatalyst is MAO.

**9.** A catalyst system as claimed in any preceding claim wherein said boron containing cocatalyst comprises an anion of formula:

$$(Z)_4B^- \qquad (III)$$

where Z is an optionally substituted phenyl derivative, said substituent being a halo-$C_{1-6}$-alkyl or halo group, preferably
triphenylcarbeniumtetrakis(pentafluorophenyl) borate,
N,N-dimethylcyclohexylammoniumtetrakis(pentafluorophenyl)borate,
N,N-dimethylbenzylammoniumtetrakis(pentafluorophenyl)borate, or
N,N-dimethylaniliniumtetrakis(pentafluorophenyl)borate.

**10.** Use in olefin polymerization of a catalyst as defined in claim 1 to 9, in a high temperature solution process at a temperature greater than 100°C for polymerizing ethylene and a C4-10 alpha-olefin comonomer to produce polyethylene with

a) a comonomer content (measured with NMR) up to 40 wt%, preferably between 5 to 40 wt%,
b) a density (measured according to ISO 1183-187) of the in the range of 0.850 $g/cm^3$ to 0.950 $g/cm^3$, preferably in the range of 0.850 $g/cm^3$ to 0.945 $g/cm^3$,
c) a Mw/Mn value (measured with GPC) of the polymers of the invention is less than 5, preferably in the range of 2.0 to 4.5. and
d) a melting points (measured with DSC according to ISO 11357-3:1999) below 130°C, preferably below 120°C.

**11.** Use according to claim 10, wherein the comonomer is butene, hexene or octene.

**12.** Process for the preparation of an ethylene copolymer comprising polymerizing ethylene and a $C_{4-10}$ alpha-olefin comonomer in a high temperature solution process at a temperature greater than 100°C in the presence of a catalyst comprising:

(i) a metallocene complex of formula (I) as defined in any of preceding claims 1 to 4;
(ii) an aluminoxane cocatalyst and
(iii) a boron containing cocatalyst.

**13.** Process according to claim 12, wherein the polymerization is performed

a) at a polymerization temperature of at least 110°C,
b) a pressure in the range of 10 to 100 bar and
c) in a liquid hydrocarbon solvent selected from the group of $C_5$-$_{12}$-hydrocarbons, which may be unsubstituted or substituted by $C_{1-4}$ alkyl group.

## Patentansprüche

**1.** Katalysatorsystem zur Herstellung von Ethylen-Copolymeren in einem Hochtemperaturlösungsverfahren, wobei das Katalysatorsystem umfasst

(i) einen Metallocen-Komplex der Formel (I)

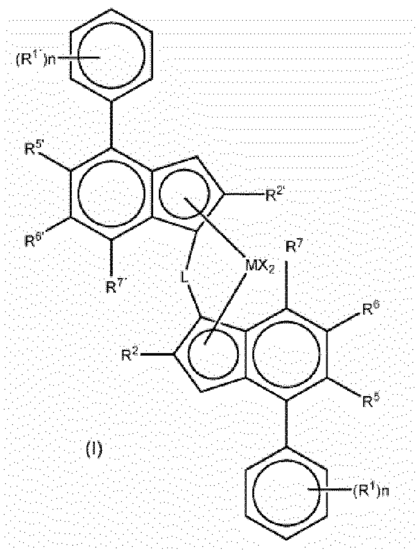

(I)

wobei

M Hf oder Zr ist,
X ein Sigmaligand ist,
L eine Brücke der Formel $-SiR^8_2-$ ist, wobei jedes $R^8$ unabhängig ein $C_1$-$C_{20}$-Hydrocarbyl, Tri($C_1$-$C_{20}$-alkyl)silyl, $C_6$-$C_{20}$-Aryl, $C_7$-$C_{20}$-Arylalkyl oder $C_7$-$C_{20}$-Alkylaryl ist,
n 0, 1 oder 2 ist,
$R^1$ und $R^{1'}$ gleich sind oder verschieden sein können und eine lineare oder verzweigte $C_1$-$C_6$-Alkylgruppe sein können,
$R^2$ und $R^{2'}$ gleich sind oder verschieden sind und eine $CH_2$-$R^9$-Gruppe, wobei $R^9$ H ist, oder eine lineare oder verzweigte $C_1$-$C_6$-Alkylgruppe sind,
$R^5$ und $R^{5'}$ verschieden sind und eines H ist und das andere eine -OR-Gruppe ist, wobei R eine $C_1$-$C_6$-Alkylgruppe ist;
$R^6$ und $R^{6'}$ gleich sind oder verschieden sind und H oder eine $C(R^{10})_3$-Gruppe sein können, wobei $R^{10}$

gleich oder verschieden ist und $R^{10}$ H oder eine lineare oder verzweigte $C_1$-$C_6$-Alkylgruppe ist; und $R^7$ und $R^{7'}$ gleich sein können oder verschieden sind und H oder eine lineare oder verzweigte $C_1$-$C_6$-Alkylgruppe sein können;

(ii) einen Aluminoxan-Cokatalysator; und
(iii) einen borhaltigen Cokatalysator.

**2.** Katalysatorsystem nach Anspruch 1, wobei in der Formel (I)
M Zr ist,
X, das gleich oder verschieden sein kann und ein Wasserstoffatom, ein Halogenatom, eine $R^{11}$-, $OR^{11}$-, $OSO_2CF_3$-, $OCOR^{11}$-, $SR^{11}$-, $NR^{11}_2$- oder $PR^{11}_2$-Gruppe ist, wobei $R^{11}$ ein linearer oder verzweigter, cyclischer oder acyclischer $C_1$-$C_{20}$-Alkyl-, $C_2$-$C_{20}$-Alkenyl-, $C_2$-$C_{20}$-Alkinyl-, $C_6$-$C_{20}$-Aryl-, $C_7$-$C_{20}$-Alkylaryl- oder $C_7$-$C_{20}$-Arylalkylrest ist; gegebenenfalls Heteroatome enthaltend, die zu Gruppen 14-16 gehören, oder $SiR^{11}_3$, $SiHR^{11}_2$ oder $SiH_2R^{11}$ ist, L eine Brücke der Formel $-SiR^8_2-$ ist, wobei beide $R^8$ die gleiche $C_1$-$C_{10}$-Hydrocarbyl- oder $C_6$-$C_{10}$-Arylgruppe sind,
$R^1$ und $R^{1'}$ gleich sind und eine lineare oder verzweigte $C_1$-$C_6$-Alkylgruppe sind,
n 0, 1 oder 2 ist, mit der Maßgabe, dass n bei mindestens einer der Phenylgruppen nicht 0 ist,
$R^2$ und $R^{2'}$ gleich sind und eine $CH_2$-$R^9$-Gruppe, wobei $R^9$ H ist, oder eine lineare oder verzweigte $C_1$-$C_4$-Alkylgruppe ist,
$R^5$ und $R^{5'}$ verschieden sind und eines H ist und das andere eine -OR-Gruppe ist, wobei R eine $C_1$-$C_4$-Alkylgruppe ist,
$R^6$ und $R^{6'}$ gleich sind oder verschieden sind und H oder eine $C(R^{10})_3$-Gruppe sein können, wobei $R^{10}$ gleich oder verschieden ist und $R^{10}$ eine lineare oder verzweigte $C_1$-$C_4$-Alkylgruppe sein kann, und
$R^7$ und $R^{7'}$ gleich sein können oder verschieden sind und H oder eine lineare oder verzweigte $C_1$-$C_4$-Alkylgruppe sein können.

**3.** Katalysatorsystem nach Anspruch 1, wobei in der Formel (I)
M Zr ist,
X unabhängig ein Wasserstoffatom, ein Halogenatom, $C_{1-6}$-Alkoxygruppe oder eine $R^{11}$-Gruppe ist, wobei $R^{11}$ eine $C_{1-6}$-Alkyl-, Phenyl- oder Benzylgruppe,
L eine Brücke der Formel $-SiR^8_2-$ ist, wobei beide $R^8$ die gleiche $C_1$-$C_{10}$-Hydrocarbyl- oder $C_6$-$C_{10}$-Arylgruppe sind,
$R^1$ und $R^{1'}$ gleich sind und eine lineare oder verzweigte $C_1$-$C_6$-Alkylgruppe sind,
n 0, 1 oder 2 ist, mit der Maßgabe, dass n bei mindestens einer der Phenylgruppen 1 ist,
$R^2$ und $R^{2'}$ gleich sind und eine $CH_2$-$R^9$-Gruppe, wobei $R^9$ H ist, oder eine lineare oder verzweigte $C_1$-$C_4$-Alkylgruppe sind,
$R^5$ und $R^{5'}$ verschieden sind und eines H ist und das andere eine OR-Gruppe ist, wobei R eine $C_1$-$C_4$-Alkylgruppe ist;
$R^6$ und $R^{6'}$ gleich sind oder verschieden sind und H oder eine $C(R^{10})_3$-Gruppe sein können, wobei $R^{10}$ gleich oder verschieden ist und $R^{10}$ eine lineare oder verzweigte $C_1$-$C_4$-Alkylgruppe sein kann, und
$R^7$ und $R^{7'}$ gleich sein können oder verschieden sind und H oder eine lineare oder verzweigte $C_1$-$C_4$-Alkylgruppe sein können.

**4.** Katalysatorsystem nach Anspruch 1, wobei das Metallocen der Formel (I) ausgewählt ist aus
racemischem Dimethylsilyl[(2-methyl-4-phenyl-5-methoxy-6-tert-butylinden-1-yl)-(2-methyl-4-phenyl-inden-1-yl)]zirkoniumdichlorid oder -dimethyl,
entweder in ihrer syn- oder anti-Konfiguration, oder
*racemischem* anti-Dimethylsilyl[ 2-methyl-4-phenyl-5-methoxy-6-*tert*-butyl-indenyl)(2-methyl-4-(4-*tert*-butyl-phenyl)indenyl)zirkoniumdichlorid.

**5.** Katalysatorsystem nach einem der vorstehenden Ansprüche, das über ein Verfahren erhalten werden kann, bei dem

(a) ein Flüssig/Flüssig-Emulsionssystem gebildet wird, wobei das Flüssig/Flüssig-Emulsionssystem eine Lösung der Katalysatorkomponenten (i) bis (iii) umfasst, die in einem Lösungsmittel dispergiert sind, um dispergierte Tröpfchen zu bilden; und
(b) durch Verfestigen der dispergierten Tröpfchen feste Teilchen gebildet werden.

**6.** Katalysatorsystem nach Anspruch 5, wobei die festen Teilchen in einem Schritt (c) vorpolymerisiert werden.

**7.** Katalysatorsystem nach einem der vorstehenden Ansprüche, bei dem es sich um ein nicht unterstütztes Katalysatorsystem handelt, das durch Inkontaktbringen des Metallocens der Formel (I) als ein Feststoff oder als eine Lösung mit dem/n zuvor in einem aromatischen Lösungsmittel aufgelösten Cokatalysator(en) erhalten werden kann, oder

durch aufeinanderfolgendes Zugeben der Katalysatorkomponenten zum Polymerisationsmedium erhalten werden kann.

**8.** Katalysatorsystem nach einem vorstehenden Anspruch, wobei der Aluminoxan-Cokatalysator MAO ist.

**9.** Katalysatorsystem nach einem vorstehenden Anspruch, wobei der borhaltige Cokatalysator ein Anion der Formel:

$(Z)_4B^-$ (III)

umfasst,

wobei Z ein gegebenenfalls substituiertes Phenylderivat ist, wobei der Substituent eine Halo-$C_{1-6}$-Alkyl- oder Halogruppe ist, vorzugsweise
Triphenylcarbeniumtetrakis(pentafluorphenyl)borat,
N,N-Dimethylcyclohexylammoniumtetrakis(pentafluorphenyl)borat,
N,N-Dimethylbenzylammoniumtetrakis(pentafluorphenyl)borat, oder
N,N-Dimethylaniliniumtetrakis(pentafluorphenyl)borat.

**10.** Verwendung eines Katalysators nach Anspruch 1 bis 9, bei der Olefinpolymerisation in einem Hochtemperaturlösungsverfahren bei einer Temperatur von mehr als 100 °C zum Polymerisieren von Ethylen und einem C4-10-alpha-Olefin-Comonomer, um Polyethylen herzustellen mit

a) einem Comonomergehalt (mit NMR gemessen) von bis zu 40 Gew.-%, vorzugsweise zwischen 5 bis 40 Gew.-%,
b) einer Dichte (nach ISO 1183-187 gemessen) des im Bereich von 0,850 $g/cm^3$ bis 0,950 $g/cm^3$, vorzugsweise im Bereich von 0,850 $g/cm^3$ bis 0,945 $g/cm^3$,
c) einem Mw/Mn-Wert (mit GPC gemessen) der erfindungsgemäßen Polymere weniger als 5 ist, vorzugsweise im Bereich von 2,0 bis 4,5., und
d) einem Schmelzpunkt (mit DSC nach ISO 11357-3:1999 gemessen) von unter 130 °C, vorzugsweise unter 120 °C.

**11.** Verwendung nach Anspruch 10, wobei das Comonomer Buten, Hexen oder Octen ist.

**12.** Verfahren zur Herstellung eines Ethylen-Copolymers, das das Polymerisieren von Ethylen und einem $C_{4-10}$-alpha-Olefin-Comonomer in einem Hochtemperaturlösungsverfahren bei einer Temperatur von mehr als 100 °C in der Gegenwart eines Katalysators umfasst, welcher umfasst:

(i) einen Metallocen-Komplex der Formel (I), nach einem der vorstehenden Ansprüche 1 bis 4;
(ii) einen Aluminoxan-Cokatalysator, und
(iii) einen borhaltigen Cokatalysator.

**13.** Verfahren nach Anspruch 12, wobei die Polymerisation durchgeführt wird

a) bei einer Polymerisationstemperatur von mindestens 110 °C,
b) einem Druck im Bereich von 10 bis 100 Bar, und
c) in einem flüssigen Kohlenwasserstofflösungsmittel, ausgewählt aus der Gruppe von $C_{5-12}$-Kohlenwasserstoffen, das unsubstituiert oder mit $C_{1-4}$-Alkylgruppe substituiert sein kann.

## Revendications

**1.** Système catalytique pour produire des copolymères d'éthylène dans un procédé en solution à haute température, le système catalytique comprenant

(i) un complexe de métallocène de formule (I)

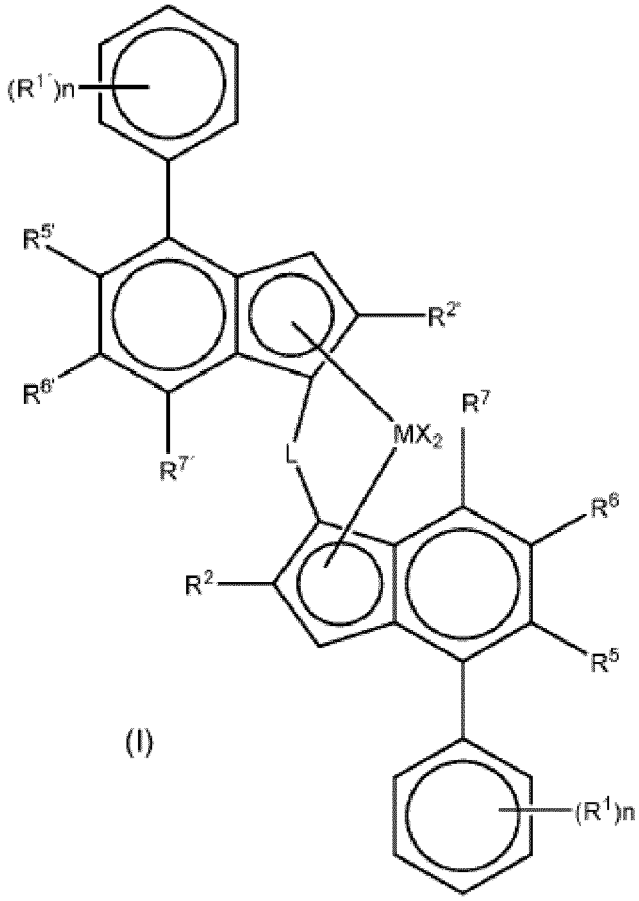

(I)

dans laquelle

M est Hf ou Zr,
X est un ligand sigma,
L est un pont de formule $-SiR^8{}_2-$, dans laquelle chaque $R^8$ est indépendamment un hydrocarbyle en $C_1$ à $C_{20}$, un tri(alkyle en $C_1$ à $C_{20}$)silyle, un aryle en $C_6$ à $C_{20}$, un arylalkyle en $C_7$ à $C_{20}$ ou un alkylaryle en $C_7$ à $C_{20}$,
n est 0, 1 ou 2,
$R^1$ et $R^{1'}$ sont identiques ou peuvent être différents et peuvent être un groupe alkyle en $C_1$ à $C_6$ linéaire ou ramifié,
$R^2$ et $R^{2'}$ sont identiques ou sont différents et sont un groupe $CH_2$-$R^9$, $R^9$ étant H ou un groupe alkyle en $C_1$ à $C_6$ linéaire ou ramifié,
$R^5$ et $R^{5'}$ sont différents et un est H et l'autre est un groupe -OR, dans lequel R est un groupe alkyle en $C_1$ à $C_6$;
$R^6$ et $R^{6'}$ sont identiques ou sont différents et peuvent être H ou un groupe $C(R^{10})_3$, les $R^{10}$ étant identiques ou différents et $R^{10}$ peut être H ou un groupe alkyle en $C_1$ à $C_6$ linéaire ou ramifié ; et
$R^7$ et $R^{7'}$ peuvent être identiques ou sont différents et peuvent être H ou un groupe alkyle en $C_1$ à $C_6$ linéaire ou ramifié ;

(ii) un cocatalyseur à base d'aluminoxane ; et
(iii) un cocatalyseur contenant du bore.

**2.** Système catalytique selon la revendication 1, dans lequel dans la formule (I)
M est Zr,
les X peuvent être identiques ou différents, et sont un atome d'hydrogène, un atome d'halogène, un groupe $R^{11}$, $OR^{11}$, $OSO_2CF_3$, $OCOR^{11}$, $SR^{11}$, $NR^{11}{}_2$ ou $PR^{11}{}_2$, dans lesquels $R^{11}$ est un radical alkyle en $C_1$ à $C_{20}$, alcényle en $C_2$ à $C_{20}$, alcynyle en $C_2$ à $C_{20}$, aryle en $C_6$ à $C_{20}$, alkylaryle en $C_7$ à $C_{20}$ ou arylalkyle en $C_7$ à $C_{20}$, linéaire ou ramifié, cyclique ou acyclique ; contenant facultativement des hétéroatomes appartenant aux groupes 14-16 ou est $SiR^{11}{}_3$, $SiHR^{11}{}_2$ ou $SiH_2R^{11}$, L est un pont de formule $-SiR^8{}_2-$, dans lequel les deux $R^8$ sont le même groupe hydrocarbyle en $C_1$ à $C_{10}$ ou aryle en $C_6$ à $C_{10}$,

$R^1$ et $R^{1'}$ sont identiques et sont un groupe alkyle en $C_1$ à $C_6$ linéaire ou ramifié,
n est 0, 1 ou 2, à condition que n ne soit pas 0 pour au moins un des groupes phényle,
$R^2$ et $R^{2'}$ sont identiques et sont un groupe $CH_2$-$R^9$, $R^9$ étant H ou un groupe alkyle en $C_1$ à $C_4$ linéaire ou ramifié,
$R^5$ et $R^{5'}$ sont différents et un est H et l'autre est un groupe -OR, dans lequel R est un groupe alkyle en $C_1$ à $C_4$,
$R^6$ et $R^{6'}$ sont identiques ou sont différents et peuvent être H ou un groupe $C(R^{10})_3$, les $R^{10}$ étant identiques ou différents et $R^{10}$ peut être un groupe alkyle en $C_1$ à $C_4$ linéaire ou ramifié, et
$R^7$ et $R^{7'}$ peuvent être identiques ou sont différents et peuvent être H ou un groupe alkyle en $C_1$ à $C_4$ linéaire ou ramifié.

**3.** Système catalytique selon la revendication 1 dans lequel dans la formule (I)
M est Zr,
X est indépendamment un atome d'hydrogène, un atome d'halogène, groupe alcoxy en $C_1$ à $C_6$ ou un groupe $R^{11}$, dans lequel $R^{11}$ un groupe alkyle en $C_1$ à $C_6$, phényle ou benzyle,
L est un pont de formule $-SiR^8_2-$, dans lequel les deux $R^8$ sont le même groupe hydrocarbyle en $C_1$ à $C_{10}$ ou aryle en $C_6$ à $C_{10}$,
$R^1$ et $R^{1'}$ sont identiques et sont un groupe alkyle en $C_1$ à $C_6$ linéaire ou ramifié,
n est 0, 1 ou 2, à condition que n soit 1 pour au moins un des groupes phényle,
$R^2$ et $R^{2'}$ sont identiques et sont un groupe $CH_2$-$R^9$, $R^9$ étant H ou un groupe alkyle en $C_1$ à $C_4$ linéaire ou ramifié,
$R^5$ et $R^{5'}$ sont différents et un est H et l'autre est un groupe OR, dans lequel R est un groupe alkyle en $C_1$ à $C_4$ ;
$R^6$ et $R^{6'}$ sont identiques ou sont différents et peuvent être H ou un groupe $C(R^{10})_3$, les $R^{10}$ étant identiques ou différents et $R^{10}$ peut être un groupe alkyle en $C_1$ à $C_4$ linéaire ou ramifié, et
$R^7$ et $R^{7'}$ peuvent être identiques ou sont différents et peuvent être H ou un groupe alkyle en $C_1$ à $C_4$ linéaire ou ramifié.

**4.** Système catalytique selon la revendication 1, dans lequel le métallocène de formule (I) est sélectionné parmi
le dichlorure ou diméthyle de diméthylsilyl[(2-méthyl-4-phényl-5-méthoxy-6-tert-butylindén-1-yl)-(2-méthyl-4-phényl-indén-1-yl)]zirconium racémique,
dans leur configuration syn ou anti, ou
le dichlorure d'anti-diméthylsilyl[2-méthyl-4-phényl-5-méthoxy-6-*tert*-butyl-indényl)(2-méthyl-4-(4-*tert*-butyl-phényl)indényl)]zirconium racémique.

**5.** Système catalytique selon l'une quelconque des revendications précédentes, pouvant être obtenu par un procédé dans lequel

(a) un système d'émulsion liquide/liquide est formé, ledit système d'émulsion liquide/liquide comprenant une solution des composants catalytiques (i) à (iii) dispersés dans un solvant de manière à former des gouttelettes dispersées ; et
(b) des particules solides sont formées en solidifiant lesdites gouttelettes dispersées.

**6.** Système catalytique selon la revendication 5, dans lequel les particules solides sont prépolymérisées dans une étape (c).

**7.** Système catalytique selon l'une quelconque des revendications précédentes, qui est un système catalytique non supporté pouvant être obtenu par la mise en contact du métallocène de formule (I) sous forme solide ou en solution avec le ou les cocatalyseurs préalablement dissous dans un solvant aromatique, ou pouvant être obtenu par l'ajout séquentiel des composants catalytiques au milieu de polymérisation.

**8.** Système catalytique selon une quelconque revendication précédente dans lequel ledit cocatalyseur à base d'aluminoxane est le MAO.

**9.** Système catalytique selon une quelconque revendication précédente dans lequel ledit cocatalyseur contenant du bore comprend un anion de formule :

$$(Z)_4B^- \qquad (III)$$

où Z est un dérivé de phényle facultativement substitué, ledit substituant étant un groupe haloalkyle en $C_1$ à $C_6$ ou halo, de préférence
le borate de triphénylcarbéniumtétrakis(pentafluorophényle),
le borate de N,N-diméthylcyclohexylammoniumtétrakis(pentafluorophényle),
le borate de N,N-diméthylbenzylammoniumtétrakis(pentafluorophényle), ou

le borate de N,N-diméthylaniliniumtétrakis(pentafluorophényle).

**10.** Utilisation dans une polymérisation d'oléfine d'un catalyseur tel que défini dans les revendications 1 à 9, dans un procédé en solution à haute température à une température supérieure à 100 °C pour polymériser de l'éthylène et un comonomère d'alpha-oléfine en C4 à C10 pour produire un polyéthylène avec

a) une teneur en comonomère (mesurée par RMN) jusqu'à 40 % en poids, de préférence entre 5 et 40 % en poids,
b) une masse volumique (mesurée conformément à l'ISO 1183-187) des dans la plage de 0,850 $g/cm^3$ à 0,950 $g/cm^3$, de préférence dans la plage de 0,850 $g/cm^3$ à 0,945 $g/cm^3$,
c) une valeur Mw/Mn (mesurée avec GPC) des polymères de l'invention est inférieure à 5, de préférence dans la plage de 2,0 à 4,5, et
d) un point de fusion (mesuré par DSC conformément à l'ISO 11357-3:1999) inférieur à 130 °C, de préférence inférieur à 120 °C.

**11.** Utilisation selon la revendication 10, dans laquelle le comonomère est le butène, l'hexène ou l'octène.

**12.** Procédé de préparation d'un copolymère d'éthylène comprenant la polymérisation de l'éthylène et d'un comonomère d'alpha-oléfine en $C_4$ à $C_{10}$ dans un procédé en solution à haute température à une température supérieure à 100 °C en présence d'un catalyseur comprenant :

(i) un complexe de métallocène de formule (I) tel que défini dans l'une quelconque des revendications 1 à 4 précédentes ;
(ii) un cocatalyseur à base d'aluminoxane et
(iii) un cocatalyseur contenant du bore.

**13.** Procédé selon la revendication 12, dans lequel la polymérisation est effectuée

a) à une température de polymérisation d'au moins 110 °C,
b) une pression dans la plage de 10 et 100 bar et
c) dans un solvant hydrocarboné liquide sélectionné dans le groupe des hydrocarbures en $C_5$ à $C_{12}$, qui peuvent être non substitués ou substitués avec groupe alkyle en $C_1$ à $C_4$.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007116034 A
- WO 2007122098 A
- EP 2532687 A
- WO 2011135004 A
- WO 2003051934 A
- WO 2012075560 A
- WO 2011135005 A
- WO 200202576 A
- WO 2012084961 A
- WO 2012001052 A
- WO 2011076780 A
- WO 03051934 A
- WO 2006069733 A
- WO 2010052263 A
- WO 2010052260 A
- WO 2010052264 A
- WO 2013007650 A1


### Non-patent literature cited in the description


- **KLIMKE, K. ; PARKINSON, M. ; PIEL, C. ; KAMINSKY, W. ; SPIESS, H.W. ; WILHELM, M.** *Macromol. Chem. Phys.,* 2006, vol. 207, 382
- **PARKINSON, M. ; KLIMKE, K. ; SPIESS, H.W. ; WILHELM, M.** *Macromol. Chem. Phys.,* 2007, vol. 208, 2128
- NMR Spectroscopy of Polymers: Innovative Strategies for Complex Macromolecules. 2011, 401
- **POLLARD, M. ; KLIMKE, K. ; GRAF, R. ; SPIESS, H.W. ; WILHELM, M. ; SPERBER, O. ; PIEL, C. ; KAMINSKY, W.** *Macromolecules,* 2004, vol. 37, 813
- **FILIP, X. ; TRIPON, C. ; FILIP, C.** *J. Mag. Resn.,* 2005, vol. 176, 239
- **GRIFFIN, J.M. ; TRIPON, C. ; SAMOSON, A. ; FILIP, C. ; BROWN, S.P.** *Mag. Res. in Chem.,* 2007, vol. 45, S1-S198
- **CASTIGNOLLES, P. ; GRAF, R. ; PARKINSON, M. ; WILHELM, M. ; GABORIEAU, M.** *Polymer,* 2009, vol. 50, 2373
- **ZHOU, Z. ; KUEMMERLE, R. ; QIU, X. ; REDWINE, D. ; CONG, R. ; TAHA, A. ; BAUGH, D. ; WINNIFORD, B.** *J. Mag. Reson.,* 2007, vol. 187, 225
- **BUSICO, V. ; CARBONNIERE, P. ; CIPULLO, R. ; PELLECCHIA, R. ; SEVERN, J. ; TALARICO, G.** *Macromol. Rapid Commun.,* 2007, vol. 28, 1128
- **J. RANDALL.** *Macromol. Sci., Rev. Macromol. Chem. Phys.,* 1989, vol. C29, 201
- **QIU, X. ; REDWINE, D. ; GOBBI, G. ; NUAMTHANOM, A. ; RINALDI, P.** *Macromolecules,* 2007, vol. 40, 6879
- **LIU, W. ; RINALDI, P. ; MCINTOSH, L. ; QUIRK, P.** *Macromolecules,* 2001, vol. 34, 4757
- *CHEMICAL ABSTRACTS,* 136040-19-2